# FASCICULE DE BREVET EUROPEEN

(11) **EP 1 197 485 B1**
(45) Date de publication et mention de la délivrance du brevet: **11.08.2004**
(21) Numéro de dépôt: 01402623.1
(22) Date de dépôt: 11.10.2001
(51) Int. Cl.: C07D 211/22, C07D 401/12, C07D 405/06, C07D 405/14, A61K 31/454, A61P 9/10

(54) **Dérivés de cyclobutène-dione et leur utilisation dans le traitement de l'arthérosclérose**
Cyclobutendion-Derivate zur Behandlung von Artherosclerose
Cyclobuten-dione derivatives for use in the treatment of artherosclerosis

(30) Priorité: 12.10.2000 FR 0013072
(43) Date de publication de la demande: 17.04.2002
(73) Titulaire: Les Laboratoires Servier, 92200 Neuilly-sur-Seine (FR)
(72) Inventeur: Peglion, Jean-Louis, 78110 Le Vesinet (FR); Vilaine, Jean-Paul, 92290 Chatenay Malabry (FR); Villeneuve, Nicole, 92500 Rueil Malmaison (FR); Thollon, Catherine, 75011 Paris (FR); Bourguignon, Marie-Pierre, 78400 Chatou (FR); Poitevin, Christophe, 75011 Paris (FR)

(56) Documents cités:
- WO-A-00/10980
- WO-A-00/35855
- BUTERA ET AL: "Design and SAR of Novel Potassium Channel Openers Targeted for Urge Urinary Incontinence.1. N-Cyanoguanidine Bioisosteres Possessing in Vivo Bladder Selectivity" J. OF MEDICINAL CHEMISTRY, vol. 43, no. 6, février 2000 (2000-02), pages 1187-1202, XP002168956

## Description

La présente invention concerne de nouveaux dérivés de cyclobutène-dione, leur procédé de préparation et les compositions pharmaceutiques qui les contiennent.

La demande de brevet WO 00/35855 décrit des dérivés de cyclobutène-dione et leur utilisation pour le traitement de l'inflammation et des maladies auto-immunes.

Les composés de la présente invention sont utiles dans le traitement des maladies ou des conditions pathologiques dans lesquelles une dysfonction endothéliale est connue comme étant un mécanisme pathogène et/ou aggravant. Ces pathologies sont : l'athérosclérose, l'existence de facteurs de risque vasculaire (dyslipidémie, diabète, hypertension artérielle systémique), les différentes formes cliniques d'ischémie myocardique ou périphérique, l'insuffisance cardiaque et les différentes formes d'hypertension artérielle pulmonaire. Ces composés sont également utiles pour le traitement de patients qui subissent une transplantation cardiaque ou une reperméabilisation vasculaire telle qu'un pontage, une thrombolyse ou une dilatation artérielle avec ou sans stent.

Une diminution de la disponibilité vasculaire en monoxyde d'azote (NO) représente le mécanisme majeur de la dysfonction endothéliale observée dans les maladies et conditions pathologiques précédemment citées et explique son rôle pathogène (*Cardiovasc. Res.,* **1999,** 43, 572 ; *Coronary. Art. Dis.* **1999,** 10, 277 ; *Coronary. Art. Dis.,* **1999,** 10, 301 ; *Coronary*. *Art. Dis.,* **1999,** 10, 287; *Coronary. Art. Dis.,* **1999,** 10, 295).

En effet, dans ces conditions pathologiques, la dysfonction endothéliale peut résulter de deux mécanismes principaux: 1) une insuffisance de production de NO liée à l'inhibition de la NO synthase endothéliale par des inhibiteurs endogènes tel l'ADMA (asymétrique diméthyle arginine) dont la concentration plasmatique augmente chez des patients présentant des facteurs de risque cardiovasculaire (*Cardiovasc. Res.,* **1999**, 43, 542 ; *Hypertension,* **1997**, 29, 242 ; *Circulation,* **1997**, 95, 2068), 2) une inactivation du NO par l'anion superoxyde (O₂⁻) dont la production est accrue dans des conditions pathologiques (*Cardiovasc. Res.,* **1999**, 43, 562 ; *Eur.J Biochem.* **1997**, 245, 541 ; *J*. *Clin. Invest.,* **1993**, 91 2546).

Le NO exerce dans les conditions normales des effets majeurs tels que: 1) la régulation de la vasomotricité artérielle par son effet vasodilatateur (*N Engl. J Med.*, **1993**, 329, 2002 ; *Nature,* **1980**, 288, 373), 2) la limitation de l'adhésion et de l'agrégation plaquettaire (*Trends Pharmacol. Sci.,* **1991,** 12, 87), 3) le contrôle de l'adhésion aux cellules endothéliales de leucocytes et de monocytes *(Proc. Natl Acad. Sci. USA*, **1991,** 88, 4651), 4) l'inhibition de la prolifération des cellules musculaires lisses vasculaires (*Cardiovasc. Res.,* **1999,** 43, 580, *Circulation,* **1993**, 87 V51)
Ceci explique que la déficience en NO au niveau de la paroi artérielle favorise des phénomènes pathologiques comme la vasoconstriction, la thrombose, l'accumulation lipidique et la prolifération des cellules musculaires lisses vasculaires.

Des expériences *in vitro* ont permis de démontrer que les composés de la présente invention permettent de limiter la dysfonction endothéliale et la diminution de disponibilité vasculaire en NO qui ont été induites par des tests impliquant les deux mécanismes physiopathologiques déjà cités : inhibition de la NO synthase endothéliale et un stress oxydatif par production d'O₂⁻.

Ainsi, grâce à leur activité pharmacologique spécifique, capable de limiter le développement de la dysfonction endothéliale, les composés de la présente invention, outre le fait qu'ils soient nouveaux, sont utiles pour prévenir le développement, l'extension et les complications des lésions athéroscléreuses notamment chez les patients présentant un facteur de risque vasculaire (dyslipidémie, diabète, hypertension artérielle), pour traiter les différentes formes cliniques d'ischémie myocardique ou périphérique, l'insuffisance cardiaque, les différentes formes d'hypertension artérielle pulmonaire. Ces composés sont également utilisés pour prévenir les complications vasculaires (spasme, thrombose, resténose, athérosclérose accélérée) chez les patients qui subissent un pontage, une dilatation vasculaire avec ou sans stent ou d'autres formes de reperméabilisation vasculaire ainsi qu'une transplantation cardiaque.

Plus spécifiquement, la présente invention concerne les composés de formule (I): 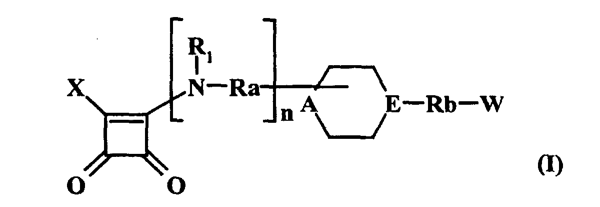 dans laquelle :
- **X**: représente :
- un hétérocycle, monocyclique ou bicyclique, saturé, partiellement insaturé, ou aromatique, de 5 à 12 chaînons, contenant de 1 à 3 hétéroatomes choisis parmi oxygène, azote, et soufre mais contenant au moins un atome d'azote, ledit hétérocycle étant lié au reste de la molécule par ledit atome d'azote, et ledit hétérocycle étant éventuellement substitué,
- ou un groupement de formule -NR₂R₃ dans laquelle R₂ représente un atome d'hydrogène ou un groupement alkyle (C₁-C₆) linéaire ou ramifié, et R₃ représente un groupement aryle éventuellement substitué, un groupement 1,3-dihydro-2H-benzimidazolyl-2-one ou un groupement alkyle (C₁-C₆) linéaire ou ramifié, substitué par un hétérocycle, monocyclique ou bicyclique, saturé, partiellement insaturé, ou aromatique, de 5 à 12 chaînons, contenant de 1 à 3 hétéroatomes choisis parmi oxygène, azote, et soufre mais contenant au moins un atome d'azote, ledit hétérocycle étant éventuellement substitué,
- **n**: représente zéro ou 1,
- **R**_{**1**}: représente un atome d'hydrogène ou un groupement alkyle (C₁-C₆) linéaire ou ramifié,
- **Ra**: représente une liaison simple ou une chaîne alkylène (C₁-C₆) linéaire ou ramifiée,
- **A**: représente un atome d'azote ou un groupement CH, mais A représente uniquement un groupement CH quand Ra représente une liaison simple, et A représente uniquement un atome d'azote quand n représente zéro,
- **E**: représente un atome d'azote ou un groupement CRe dans lequel Re représente, soit un atome d'hydrogène, soit une liaison avec un atome de carbone de W, étant entendu que l'un au moins des groupements A ou E représente un atome d'azote,
- **Rb**: représente une liaison simple ou une chaîne alkylène (C₁-C₆) linéaire ou ramifiée, dont un des atomes de carbone est éventuellement remplacé par un atome d'oxygène ou un atome de soufre,
- **W**: représente un groupement aryle ou hétéroaryle, chacun de ces groupements étant éventuellement substitué,
leurs isomères optiques, leurs hydrates, leurs solvates ainsi que leurs sels d'addition à un acide pharmaceutiquement acceptable,
étant entendu que :
- par groupement aryle, on comprend un groupement choisi parmi phényle, biphényle, bicyclo[4.2.0]octa-1,3,5-triényle, naphtyle, dihydronaphtyle, tétrahydro-naphtyle, indanyle et indényle,
- par groupement hétéroaryle, on comprend un groupement aryle tel que défini précédemment contenant de 1 à 3 hétéroatomes, identiques ou différents, choisis parmi oxygène, azote et soufre,
- par "éventuellement substitué" affecté aux termes "hétérocycle", "aryle" ou "hétéroaryle", on entend éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, mercapto, alkylthio (C₁-C₆) linéaire ou ramifié, trihalogénoalkyle (C₁-C₆) linéaire ou ramifié, cyano, nitro, amino, alkylamino (C₁-C₆) linéaire ou ramifié, dialkylamino (C₁-C₆) linéaire ou ramifié et méthylènedioxy,
- par isomères optiques, on comprend les énantiomères et les diastéréoisomères.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique.

D'une façon préférentielle, les composés de l'invention sont les composés de formule (IA) : 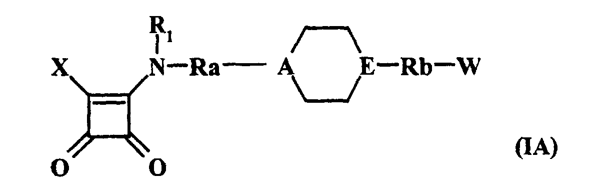 dans laquelle X, R₁, Ra, A, E, Rb et W sont tels que définis dans la formule (I).

Selon une variante avantageuse de l'invention, les composés préférés sont les composés de formule (I) dans laquelle X représente un hétérocycle tel que défini dans la formule (I).

Selon une autre variante avantageuse, les composés préférés de l'invention sont les composés de formule (I) dans laquelle X représente un groupement de formule -NR₂R₃ tel que défini dans la formule (I).

D'une façon particulièrement avantageuse, les composés préférés de l'invention sont les composés de formule (I) dans laquelle X représente un groupement 1-indolinyle ou 2,3-dihydro-1H-pyrrolo[2,3-c]pyridin-1-yle, chacun de ces groupements étant éventuellement substitué par un ou plusieurs groupements tel que définis dans la formule (I).

D'une autre façon particulièrement avantageuse, les composés préférés de l'invention sont les composés de formule (I) dans laquelle X représente un groupement de formule -NR₂R₃ dans laquelle R₂ représente un atome d'hydrogène et R₃ représente un groupement phényle éventuellement substitué.

Selon une variante intéressante de l'invention, les composés préférés de l'invention sont les composés de formule (I) dans laquelle W représente un groupement hétéroaryle, et d'une façon avantageuse, W représente un groupement benzofuryle ou indolyle.

Selon une autre variante intéressante de l'invention, les composés préférés de l'invention sont les composés de formule (I) dans laquelle -Rb-W forment ensemble un groupement aryloxyalkyle (C₁-C₅) linéaire ou ramifié, la partie aryle étant éventuellement substituée.

Les composés préférés de l'invention sont le :
- 3-anilino-4-({2-[4-(phénoxyméthyl)-1-pipéridinyl]éthyl}amino)-3-cyclobutène-1,2-dione et son méthanesulfonate,
- 3-(4-chloroanilino)-4-({2-[4-(phénoxyméthyl)-1-pipéridinyl]éthyl}amino)-3-cyclobutène-1,2-dione et son méthanesulfonate,
- 3-(2,3-dihydro-1*H*-indol-1-yl)-4-({2-[4-(phénoxyméthyl)-1-pipéridinyl]éthyl}amino)-3-cyclobutène-1,2-dione et son méthanesulfonate,
- 3-({1-[2-(1-benzofuran-3-yl)éthyl]-4-pipéridinyl}amino)-4-(4-fluoroanilino)-3-cyclobutène-1,2-dione et son méthanesulfonate,
- 4-{[2-({1-[2-(1-benzofuran-3-yl)éthyl]-4-pipéridinyl}amino)-3,4-dioxo-1-cyclobutèn-1-yl]amino}benzonitrile et son méthanesulfonate,
- 3-[(2-{4-[2-(4-fluorophénoxy)éthyl]-1-pipéridinyl}éthyl)amino]-4-[(2-oxo-2,3-dihydro - 1*H*-benzimidazol-5-yl)amino]-3-cyclobutène-1,2-dione et son méthanesulfonate
- 3-(5-méthoxy-2,3-dihydro-1*H*-pyrrolo[2,3-c]pyridin-1-yl)-4-({2-[4-(phénoxyméthyl)-1-pipéridinyl]éthyl}amino)-3-cyclobutène-1,2-dione et son dichlorhydrate
- 3-[(2-{4-[2-(4-fluorophénoxy)éthyl]-1-pipéridinyl}éthyl)amino]-4-(5-méthoxy-2,3-dihydro-1*H*-pyrrolo[2,3-c]pyridin-1-yl)-3-cyclobutène-1,2-dione et son dichlorhydrate
- 3-[(2-{4-[2-(4-fluorophénoxy)éthyl]-1-pipéridinyl}éthyl)amino]-4-(4,5,6-triméthoxy-2,3-dihydro-1*H*-indol-1-yl)-3-cyclobutène-1,2-dione et son méthanesulfonate
- 3-(6-chloro-2,3-dihydro-1*H*-indol-1-yl)-4-[(2-{4-[2-(4-fluorophénoxy)-éthyl]-1-pipéridinyl}éthyl)amino]-3-cyclobutène-1,2-dione et son méthane sulfonate
- 3-(2,3-dihydro-1*H*-indol-1-yl)-4-[({1-[2-(1*H*-indol-3-yl)éthyl]-3-pipéridinyl}méthyl) amino]-3-cyclobutène-1,2-dione et son chlorhydrate
- 3-(5,6-diméthoxy-2,3-dihydro-1*H*-indol-1-yl)-4-[(2-{4-[2-(4-fluorophénoxy)éthyl]-1-pipéridinyl}éthyl)amino]-3-cyclobutène-1,2-dione et son méthanesulfonate
- 3-[(2-{4-[2-(4-fluorophénoxy)éthyl]-1-pipéridinyl}éthyl)amino]-4-(6-méthoxy-2,3-dihydro-1*H*-indol-1-yl)-3-cyclobutène-1,2-dione et son méthanesulfonate, et le
- 3-(4,5-diméthoxy-2,3-dihydro-1*H*-indol-1-yl)-4-[(2-{4-[2-(4-fluorophénoxy)éthyl]-1-pipéridinyl}éthyl)amino]-3-cyclobutène-1,2-dione et son méthanesulfonate.

Les isomères, les hydrates, les solvates ainsi que les sels d'addition à un acide pharmaceutiquement acceptable des composés préférés de l'invention font partie intégrante de l'invention.

La présente invention s'étend également au procédé de préparation des composés de formule (I), caractérisé en ce que soit utilisé comme produit de départ, un composé de formule (II) : 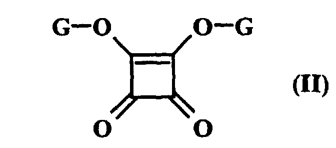 dans laquelle G représente un groupement alkyle (C₁-C₄) linéaire ou ramifié,
composé de formule (II) qui est mis à réagir avec une amine de formule (III) :

**X - H (III)**

dans laquelle X est tel que défini dans la formule (I),
pour conduire au composé de formule (IV) : 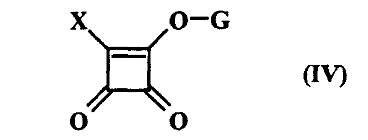 dans laquelle X et G sont tels que définis précédemment,
composé de formule (IV) qui est traité avec un composé de formule (V) : 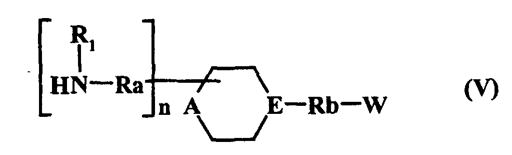 dans laquelle n, R₁, Ra, Rb, A, E et W sont tels que définis dans la formule (I),
pour conduire aux composés de formule (I) telle que définie : 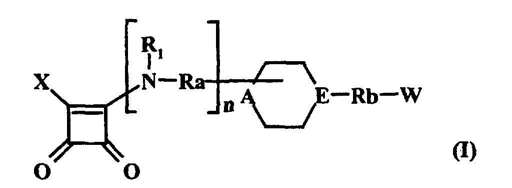 composés de formule (I) que l'on purifie, le cas échéant, selon une technique classique de purification, qui peuvent, si on le désire, être séparés en leurs différents isomères optiques selon une technique classique de séparation et que l'on transforme, si on le souhaite, en leurs sels d'addition à un acide pharmaceutiquement acceptable.

Les composés de formule (II), (III) et (V) sont soit des composés commerciaux, soit obtenus selon des méthodes classiques de la synthèse organique.

Les composés de la présente invention sont utiles dans le traitement des maladies ou des conditions pathologiques dans lesquelles une dysfonction endothéliale est connue. De ce fait, grâce à leur activité pharmacologique spécifique, les composés de l'invention sont utiles pour prévenir le développement, l'extension et les complications des lésions athéroscléreuses, dans le traitement de l'ischémie myocardique ou périphérique, de l'insuffisance cardiaque, de l'hypertension artérielle pulmonaire, pour la prévention des complications vasculaires après pontage vasculaire, dilatation vasculaire, repennéabilisation vasculaire et transplantation cardiaque.

La présente invention a également pour objet les compositions pharmaceutiques contenant comme principe actif au moins un composé de formule (I), ses isomères optiques, ses hydrates, ses solvates, ses sels d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature et la sévérité de l'affection, et la prise de traitements éventuels associés et s'échelonne de 1 mg à 200 mg en une ou plusieurs prises par jour.
Les exemples suivants illustrent l'invention mais ne la limitent en aucune façon. Les produits de départ utilisés sont des produits connus ou préparés selon des modes opératoires connus. Les différentes préparations conduisent à des intermédiaires de synthèse utiles pour la préparation des composés de l'invention.

Les structures des composés décrits dans les exemples ont été déterminées selon les techniques spectrophotométriques usuelles (infrarouge, résonance magnétique nucléaire, spectrométrie de masse, ...).

Les points de fusion ont été déterminés soit à la platine chauffante de Kofler (K.), soit à la platine chauffante sous microscope (M.K.).

### EXEMPLE 1: 3-Anilino-4-({2-[4-(phénoxyméthyl)-1-pipéridinyl]éthyl}amino)-3-cyclobutène-1,2-dione et son méthanesulfonate

### Stade 1: 4-Anilino-3-éthoxy-3-cyclobutène-1,2-dione

0,01 mol d'aniline est additionnée à une solution de 0,01 mol de 3,4-diéthoxy-3-cyclobutène-1,2-dione dans 30 ml d'éthanol anhydre. Le mélange est porté au reflux pendant 12 heures et le précipité formé est filtré à chaud. Le résidu obtenu est concrétisé en présence d'éther permettant d'isoler le produit attendu.
**Point de fusion (K): 110°C**

### Stade 2: 3-Anilino-4-({2-[4-(phénoxyméthyl)-1-pipéridinyl]éthyl}amino)-3-cyclobutène-1,2-dione et son méthanesulfonate

0,01 mol du produit obtenu au stade 1 et 0,01 mol de 2-[4-(phénoxyméthyl)-1-pipéridinyl]éthylamine dans 66 ml d'éthanol anhydre sont portés à reflux pendant 18 heures. Le précipité obtenu est filtré à chaud et séché pour conduire au produit attendu que l'on transforme en son sel de méthanesulfonate par action d'une solution de 1,5 équivalents d'acide méthanesulfonique dans du méthanol à reflux pendant 2 heures.
**Point de fusion (M.K.) : 258-262 °C**

### EXEMPLE 2 : 3-(4-Fluoroanilino)-4-({2-[4-(phénoxyméthyl)-1-pipéridinyl] éthyl}amino)-3-cyclobutène-1,2-dione et son méthanesulfonate

### Stade 1 : 3-Ethoxy-4-(4-fluoroanilino)-3-cyclobutène-1,2-dione

Le produit est obtenu selon le procédé décrit dans le stade 1 de l'exemple 1 en utilisant la p-fluoroaniline à la place de l'aniline.
**Point de fusion (K) : 180°C**

### Stade 2: 3-(4-Fluoroanilino)-4-({2-[4-(phénoxyméthyl)-1-pipéridinyl]éthyl}amino)-3-cyclobutène-1,2-dione et son méthanesulfonate

Le produit est obtenu selon le procédé décrit dans le stade 2 de l'exemple 1 en utilisant le composé décrit dans le stade précédent.
**Point de fusion (M.K.) : 218-221°C**

### EXEMPLE 3 : 3-(4-Chloroanilino)-4-({2-[4-(phénoxyméthyl)-1-pipéridinyl] éthyl}amino)-3-cyclobutène-1,2-dione et son méthanesulfonate

### Stade 1 : 4-(4-Chloroanilino)-3-éthoxy-3-cyclobutène-1,2-dione

Le produit est obtenu selon le procédé décrit dans le stade 1 de l'exemple 1 en utilisant la p-chloroaniline à la place de l'aniline.
**Point de fusion (K) : 149°C**

### Stade 2: 3-(4-Chloroanilino)-4-({2-[4-(phénoxyméthyl)-1-pipéridinyl]éthyl}amino)-3-cyclobutène-1,2-dione et son méthanesulfonate

Le produit est obtenu selon le procédé décrit dans le stade 2 de l'exemple 1 en utilisant le composé décrit dans le stade précédent.
**Point de fusion (M.K.) : 243-246°C**

### EXEMPLE 4: 4-{[3,4-Dioxo-2-({2-[4-(phénoxyméthyl)-1-pipéridinyl]éthyl}amino)-1-cyclobutèn-1-yl]amino}benzonitrile et son méthanesulfonate

### Stade 1: 4-(4-Cyanoanilino)-3-éthoxy-3-cyclobutène-1,2-dione

Le produit est obtenu selon le procédé décrit dans le stade 1 de l'exemple 1 en utilisant la p-cyanoaniline à la place de l'aniline.
**Point de fusion (K) : 230°C**

### Stade 2: 4-{[3,4-Dioxo-2-({2-[4-(phénoxyméthyl)-1-pipéridinyl]éthyl}amino)-1-cyclobutèn-1-yl]amino}benzonitrile et son méthanesulfonate

Le produit est obtenu selon le procédé décrit dans le stade 2 de l'exemple 1 en utilisant le composé décrit dans le stade précédent.
**Point de fusion (M.K.) : 295-299°C**

### EXEMPLE 5: 3-(2,3-Dihydro-1H-indol-1-yl)-4-({2-[4-(phénoxyméthyl)-1-pipéridinyl]éthyl}amino)-3-cyclobutène-1,2-dione et son méthanesulfonate

### Stade 1: 3-Ethoxy-4-(indolin-1-yl)-3-cyclobutène-1,2-dione

Le produit est obtenu selon le procédé décrit dans le stade 1 de l'exemple 1 en utilisant l'indoline à la place de l'aniline.
**Point de fusion (K) : 204°C**

### Stade 2: 3-(2,3-Dihydro-1H-indol-1-yl)-4-({2-[4-(phénoxyméthyl)-1-pipéridinyl] éthyl}amino)-3-cyclobutène-1,2-dione et son méthanesulfonate

Le produit est obtenu selon le procédé décrit dans le stade 2 de l'exemple 1 en utilisant le composé décrit dans le stade précédent.
**Point de fusion (M.K.) : 209-213°C**

### EXEMPLE 6: 3-Anilino-4-({1-[2-(1-benzofuran-3-yl)éthyl]-4-pipéridinyl}amino)-3-cyclobutène-1,2-dione et son méthanesulfonate

Le produit est obtenu selon le procédé du stade 2 de l'exemple 1 mais en utilisant comme substrat le produit du stade 1 de l'exemple 1 et la 1-[2-(1-benzofuran-3-yl)éthyl]4-pipéridinylamine.
**Point de fusion (M.K.) : 276-280°C**

### EXEMPLE 7: 3-({1-[2-(1-Benzofuran-3-yl)éthyl]-4-pipéridinyl}amino)-4-(4-fluoroanilino)-3-cyclobutène-1,2-dione et son méthanesulfonate

Le produit est obtenu selon le procédé de l'exemple 6 en utilisant le produit du stade 1 de l'exemple 2 à la place du produit du stade 1 de l'exemple 1.
**Point de fusion (M.K.) : 266 -271°C**

### EXEMPLE 8: 3-({1-[2-(1-Benzofuran-3-yl)éthyl]-4-pipéridinyl}amino)-4-(4-chloroanilino)-3-cyclobutène-1,2-dione et son méthanesulfonate

Le produit est obtenu selon le procédé de l'exemple 6 en utilisant le produit du stade 1 de l'exemple 3 à la place du produit du stade 1 de l'exemple 1.
**Point de fusion (M.K.) : 283 -287°C**

### EXEMPLE 9 : 4-{[2-({1-[2-(1-Benzofuran-3-yl)éthyl]-4-pipéridinyl}amino)-3,4-dioxo-1-cyclobutèn-1-yl]amino}benzonitrile et son méthanesulfonate

Le produit est obtenu selon le procédé de l'exemple 6 en utilisant le produit du stade 1 de l'exemple 4 à la place du produit du stade 1 de l'exemple 1.
**Point de fusion (M.K.) : 295 - 299°C**

### EXEMPLE 10: 3-({1-[2-(1-benzofuran-3-yl)éthyl]-4-pipéridinyl}amino)-4-(2,3-dihydro-1H-indol-1-yl)-3-cyclobutène-1,2-dione et son méthanesulfonate

Le produit est obtenu selon le procédé de l'exemple 6 en utilisant le produit du stade 1 de l'exemple 5 à la place du produit du stade 1 de l'exemple 1.
**Point de fusion (M.K.) : 265-270°C**

### EXEMPLE 11 : 3-(4-Chloroanilino)-4-[(2-{4-[2-(4-fluorophénoxy)éthyl]-1-pipéridinyl} éthyl)amino]-3-cyclobutène-1,2-dione et son méthanesulfonate

Le produit est obtenu selon le procédé de l'exemple 1 en utilisant comme substrat au stade 1 la *p*-chloroaniline et au stade 2 la 2-{4-[2-(4-fluorophénoxy)éthyl]-1-pipéridinyl} éthylamine.
**Point de fusion : 215-219°C**

### EXEMPLE 12 : 3-[(2-{4-[2-(4-Fluorophénoxy)éthyl]-1-pipéridinyl}éthyl)amino]-4-(4-nitroanilino)-3-cyclobutène-1,2-dione et son méthanesulfonate

Le produit est obtenu selon le procédé de l'exemple 1 en utilisant comme substrat au stade 1 la *p*-nitroaniline et au stade 2 la 2-{4-[2-(4-fluorophénoxy)éthyl]-1-pipéridinyl} éthylamine.
**Point de fusion : 223-227°C**

### EXEMPLE 13: 3-[(2-{4-[2-(4-Fluorophénoxy)éthyl]-1-pipéridinyl}éthyl)amino]-4-(5-nitro-2,3-dihydro-1H-indol-1-yl)-3-cyclobutène-1,2-dione et son méthanesulfonate

Le produit est obtenu selon le procédé de l'exemple 1 en utilisant comme substrat au stade 1 la 5-nitroindoline et au stade 2 la 2-{4-[2-(4-fluorophénoxy)éthyl]-1-pipéridinyl} éthylamine.
**Point de fusion : 232-235°C**

### EXEMPLE 14: 3-[(2-Oxo-2,3-dihydro-1H-benzimidazol-5-yl)amino]-4-({2-[4-(phénoxy méthyl)-1-pipéridinyl]éthyl}amino)-3-cyclobutène-1,2-dione et son méthanesulfonate

Le produit est obtenu selon le procédé de l'exemple 1 en utilisant comme substrat au stade 1 le 5-amino-1,3-dihydro-2*H*-benzimidazol-2-one.
**Point de fusion : 176-180°C**

### EXEMPLE 15: 3-[(2-{4-[2-(4-Fluorophénoxy)éthyl]-1-pipéridinyl}éthyl)amino]-4-[(2-oxo-2,3-dihydro-1H-benzimidazol-5-yl)amino]-3-cyclobutène-1,2-dione et son méthanesulfonate

Le produit est obtenu selon le procédé de l'exemple 1 en utilisant comme substrat au stade 1 le 5-amino-1,3-dihydro-2*H*-benzimidazol-2-one et au stade 2 la 2-{4-[2-(4-fluorophénoxy)éthyl]-1-pipéridinyl}éthylamine.
**Point de fusion : 184-188°C**

### EXEMPLE 16 : 3-(6,7-Dihydro-5H-[1,3]dioxolo[4,5-f]indol-5-yl)-4-({2-[4-(phénoxy méthyl)-1-pipéridinyl]éthyl}amino)-3-cyclobutène-1,2-dione et son méthanesulfonate

Le produit est obtenu selon le procédé de l'exemple 1 en utilisant comme substrat au stade 1 le 6,7-dihydro-5*H*-[1,3]dioxolo[4,5-*f*]indole.
**Point de fusion : 193-196°C**

### EXEMPLE 17 : 3-(5-Méthoxy-2,3-dihydro-1H-pyrrolo[2,3-c]pyridin-1-yl)-4-({2-[4-(phénoxyméthyl)-1-pipéridinyl]éthyl}amino)-3-cyclobutène-1,2-dione et son dichlorhydrate

Le produit est obtenu selon le procédé de l'exemple 1 en utilisant comme substrat au stade 1 le 5-méthoxy-2,3-dihydro-1*H*-pyrrolo[2,3-c]pyridine.
**Point de fusion : 190-195°C**

### EXEMPLE 18 : 3-[(2-{4-[2-(4-Fluorophénoxy)éthyl]-1-pipéridinyl}éthyl)amino]-4-(5-méthoxy-2,3-dihydro-1H-pyrrolo[2,3-c]pyridin-1-yl)-3-cyclobutène-1,2-dione et son dichlorhydrate

Le produit est obtenu selon le procédé de l'exemple 1 en utilisant comme substrat au stade 1 le 5-méthoxy-2,3-dihydro-1*H*-pyrrolo[2,3-c]pyridine et au stade 2 la 2-{4-[2-(4-fluorophénoxy)éthyl]-1-pipéridinyl}éthylamine.
**Point de fusion : 224-228°C**

### EXEMPLE 19: 3-(5-Fluoro-2,3-dihydro-1H-indol-1-yl)-4-[(2-{4-[2-(4-fluorophénoxy)-éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 5-fluoroindoline et au stade 2 la 2-{4-[2-(4-fluorophénoxy)éthyl]-1-pipéridinyl} éthylamine.
**Point de fusion : 166-170°C**

### EXEMPLE 20 : 3-[(2-{4-[2-(4-Fluorophénoxy)éthyl]-1-pipéridinyl}éthyl)amino]-4-(4,5,6-triméthoxy-2,3-dihydro-1H-indol-1-yl)-3-cyclobutène-1,2-dione et son méthanesulfonate

Le produit est obtenu selon le procédé de l'exemple 1 en utilisant comme substrat au stade 1 la 4,5,6-triméthoxyindoline et au stade 2 la 2-{4-[2-(4-fluorophénoxy)éthyl]-1-pipéridinyl}éthylamine.
**Point de fusion : 176-180°C**

### EXEMPLE 21 : 3-(2,3-Dihydro-1H-indol-1-yl)-4-[(2-{4-[2-(4-fluorophénoxy)éthyl]-1-pipéridinyl}éthyl)amino]-3-cyclobutène-1,2-dione et son méthane sulfonate

Le produit est obtenu selon le procédé de l'exemple 1 en utilisant comme substrat au stade 1 l'indoline et au stade 2 la 2-{4-[2-(4-fluorophénoxy)éthyl]-1-pipéridinyl}éthylamine.
**Point de fusion : 182-186°C**

### EXEMPLE 22 : 3-(6-Chloro-2,3-dihydro-1H-indol-1-yl)-4-[(2-{4-[2-(4-fluorophénoxy)-éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 6-chloroindoline et au stade 2 la 2-{4-[2-(4-fluorophénoxy)éthyl]-1-pipéridinyl} éthylamine.
**Point de fusion : 182-186°C**

### EXEMPLE 23 : 3-[(2-{4-[2-(4-Fluorophénoxy)éthyl]-1-pipéridinyl}éthyl)amino]-4-(5-méthoxy-2,3-dihydro-1H-indol-1-yl)-3-cyclobutène-1,2-dione et son méthanesulfonate

Le produit est obtenu selon le procédé de l'exemple 1 en utilisant comme substrat au stade 1 la 5-méthoxyindoline et au stade 2 la 2-{4-[2-(4-fluorophénoxy)éthyl]-1-pipéridinyl}éthylamine.
**Point de fusion : 182-185°C**

### EXEMPLE 24 : 3-[(1-{3-[2-(3,4-Difluorophényl)éthoxy]propyl}-4-pipéridinyl)amino]-4-(2,3-dihydro-1H-indol-1-yl)-3-cyclobutène-1,2-dione et son chlorhydrate

Le produit est obtenu selon le procédé de l'exemple 1 en utilisant comme substrat au stade 1 l'indoline et au stade 2 la 1-{3-[2-(3,4-difluorophényl)éthoxy]propyl}-4-pipéridinamine.
**Point de fusion : 250-254°C**

### EXEMPLE 25 : 3-(5-Chloro-2,3-dihydro-1H-indol-1-yl)-4-[(2-{4-[2-(4-fluorophénoxy)-éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 5-chloroindoline et au stade 2 la 2-{4-[2-(4-fluorophénoxy)éthyl]-1-pipéridinyl} éthylamine.
**Point de fusion : 167-171°C**

### EXEMPLE 26 : 3-(2,3-Dihydro-1H-indol-1-yl)-4-{[2-(4-{[(4-fluorobenzyl)oxyl]méthyl}-1-pipéridinyl)éthyl]amino}-3-cyclobutène-1,2-dione et son chlorhydrate

Le produit est obtenu selon le procédé de l'exemple 1 en utilisant comme substrat au stade 1 l'indoline et au stade 2 la 2-(4-{[(4-fluorobenzyl)oxy]méthyl}-1-pipéridinyl)éthanamine.
**Point de fusion : 230-235°C**

### EXEMPLE 27 : 3-(2,3-Dihydro-1H-indol-1-yl)-4-[({1-[2-(1H-indol-3-yl)éthyl]-3-pipéridinyl}méthyl)amino]-3-cyclobutène-1,2-dione et son chlorhydrate

Le produit est obtenu selon le procédé de l'exemple 1 en utilisant comme substrat au stade 1 l'indoline et au stade 2 la {1-[2-(1*H*-indol-3-yl)éthyl]-3-pipéridinyl}méthanamine.
**Point de fusion : 237-242°C**

### EXEMPLE 28 : 3-(2,3-Dihydro-1H-indol-1-yl)-4-[({1-[2-(3,4-diméthoxyphényl)éthyl]-3-pipéridinyl}méthyl)amino]-3-cyclobutène-1,2-dione et son chlorhydrate

Le produit est obtenu selon le procédé de l'exemple 1 en utilisant comme substrat au stade 1 l'indoline et au stade 2 la {1-[2-(3,4-diméthoxyphényl)éthyl]-3-pipéridinyl} méthanamine.
**Point de fusion : 228-232°C**

### EXEMPLE 29 : 3-(5,6-Diméthoxy-2,3-dihydro-1H-indol-1-yl)-4-[(2-{4-[2-(4-fluoro phénoxy)éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 5,6-diméthoxyindoline et au stade 2 la 2-{4-[2-(4-fluorophénoxy)éthyl]-1-pipéridinyl}éthylamine.
**Point de fusion : 102-106°C**

### EXEMPLE 30 : 3-({1-[2-(1-Benzofuran-3-yl)éthyl]-4-pipéridinyl}amino)-4-(5-méthoxy-2,3-dihydro-1H-pyrrolo[2,3-c]pyridin-1-yl)-3-cyclobutène-1,2-dione et son chlorhydrate

Le produit est obtenu selon le procédé de l'exemple 1 en utilisant comme substrat au stade 1 le 5-méthoxy-2,3-dihydro-1*H*-pyrrolo[2,3-c]pyridine et au stade 2 la 1-[2-(1-benzofuran-3-yl)éthyl]-4-pipéridinamine
**Point de fusion : 278-288°C**

### EXEMPLE 31 : 3-[(2-{4-[2-(4-Fluorophénoxy)éthyl]-1-pipéridinyl}éthyl)amino]-4-(4-méthoxy-2,3-dihydro-1H-indol-1-yl)-3-cyclobutène-1,2-dione et son méthanesulfonate

Le produit est obtenu selon le procédé de l'exemple 1 en utilisant comme substrat au stade 1 la 4-méthoxyindoline et au stade 2 la 2-{4-[2-(4-fluorophénoxy)éthyl]-1-pipéridinyl} éthylamine.
**Point de fusion : 240-244°C**

### EXEMPLE 32 : 3-[(2-{4-[2-(4-Fluorophénoxy)éthyl]-1-pipéridinyl}éthyl)amino]-4-(6-méthoxy-2,3-dihydro-1H-indol-1-yl)-3-cyclobutène-1,2-dione et son méthanesulfonate

Le produit est obtenu selon le procédé de l'exemple 1 en utilisant comme substrat au stade 1 la 6-méthoxyindoline et au stade 2 la 2-{4-[2-(4-fluorophénoxy)éthyl]-1-pipéridinyl} éthylamine.
**Point de fusion : 178-182°C**

### EXEMPLE 33 : 3-(5-Méthoxy-2,3-dihydro-1H-pyrrolo[2,3-c]pyridin-1-yl)-4-({2-[4-(2,3,4-triméthoxybenzyl)-1-pipérazinyl]éthyl}amino)-3-cyclobutène-1,2-dione et son trichlorhydrate

Le produit est obtenu selon le procédé de l'exemple 1 en utilisant comme substrat au stade 1 la 5-méthoxy-2,3-dihydro-1*H*-pyrrolo[2,3-*c*]pyridine et au stade 2 la 2-[4-(2,3,4-triméthoxybenzyl)-1-pipérazinyl]éthylamine.
**Point de fusion : 181-183°C**

### EXEMPLE 34 : 3-(5-Méthoxy-2,3-dihydro-1H-pyrrolo[2,3-c]pyridin-1-yl)-4-[4-(2,3,4-triméthoxybenzyl)-1-pipérazinyl]-3-cyclobutène-1,2-dione et son fumarate

Le produit est obtenu selon le procédé de l'exemple 1 en utilisant comme substrat au stade 1 la 5-méthoxy-2,3-dihydro-1*H*-pyrrolo[2,3-c]pyridine et au stade 2 la 4-(2,3,4-triméthoxybenzyl)-1-pipérazine.
Le produit attendu est transformé en son fumarate par action d'une solution d'acide fumarique.
**Point de fusion : 190-195°C**

### EXEMPLE 35 : 3-[2-{4-[2-(4-Fluorophénoxy)éthyl]-1-pipéridinyl}éthyl)amino]-4-{[(1-méthoxy-cyclobuta[c]pyridin-6-yl)-méthyl]-amino}-3-cyclobutène-1,2-dione et son hémifumarate

Le produit est obtenu selon le procédé de l'exemple 1 en utilisant comme substrat au stade 1 la 6-aminométhyl-1-méthoxy-cyclobuta[c]pyridine et au stade 2 la 2-{4-[2-(4-fluorophénoxy)-éthyl]-1-pipéridinyl}-éthylamine.
Le produit attendu est transformé en son hémifumarate par action d'une solution d'acide fumarique.
**Point de fusion : 190-197°C**

### EXEMPLE 36 : 3-(5-Méthoxy-2,3-dihydro-1H-pyrrolo[2,3-c]pyridin-1-yl)-4-{[2-(spiro[2,3-dihydro-1-benzofurane-3:4'-pipéridin-1-yl])-éthyl]-amino}-3-cyclobutène-1,2-dione et son sesquichlorhydrate

Le produit est obtenu selon le procédé de l'exemple 1 en utilisant comme substrat au stade 1 la 5-méthoxy-2,3-dihydro-1*H*-pyrrolo[2,3-c]pyridine et au stade 2 la 1-(2-aminoéthyl)-spiro[2,3-dihydro-1-benzofurane-3:4'-pipéridine].
Le produit attendu est transformé en son sesquichlorhydrate par action d'une solution d'acide chlorhydrique.
**Point de fusion 275-280°C**

### EXEMPLE 37: 3-(4,5-Diméthoxy-2,3-dihydro-1H-indol-1-yl)-4-[(2-{4-[2-(4-fluoro phénoxy)éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 4,5-diméthoxyindoline et au stade 2 la 2-{4-[2-(4-fluorophénoxy)éthyl]-1-pipéridinyl} éthylamine.
**Point de fusion : 202-207°C**

### EXEMPLE 38 : 3-(4,5-Diméthoxy-2,3-dihydro-1H-indol-1-yl)-4-[(2-{4-(phénoxyméthyl)-1-pipéridinyl}-éthyl-)amino]-3-cyclobutène-1,2-dione et son méthanesulfonate

Le produit est obtenu selon le procédé de l'exemple 1 en utilisant comme substrat au stade 1 la 4,5-diméthoxyindoline.
**Point de fusion : 209-213°C**

### EXEMPLE 39 : 3-(4,6-Diméthoxy-2,3-dihydro-1H-indol-1-yl)-4-[(2-{4-[2-(4-fluoro phénoxy)éthyl]-1-pipéridinyl}éthyl)amino]-3-cyclobutène-1,2-dione et son méthanesulfonate

Le produit est obtenu selon le procédé de l'exemple 1 en utilisant comme substrat au stade 1 la 4,6-diméthoxyindoline et au stade 2 la 2-{4-[2-(4-fluorophénoxy)éthyl]-1-pipéridinyl} éthylamine.
**Point de fusion : 175-180°C**

### EXEMPLE 40 : 3-(4,6-Diméthoxy-2,3-dihydro-1H-indol-1-yl)-4-[(2-{4-(phénoxyméthyl)-1-pipéridinyl}-éthyl-)amino]-3-cyclobutène-1,2-dione et son méthanesulfonate

Le produit est obtenu selon le procédé de l'exemple 1 en utilisant comme substrat au stade 1 la 4,6-diméthoxyindoline.
**Point de fusion : 236-240°C**

### ETUDE PHARMACOLOGIOUE DES COMPOSES DE L'INVENTION

Dans des conditions standard *in vitro*, la relaxation à l'acétylcholine (ACh) d'anneaux aortiques, totalement dépendante de la présence de l'endothélium, est le reflet de la production de NO (stimulée par l'ACh) qui en diffusant au niveau des cellules musculaires lisses entraîne la relaxation artérielle (*Nature*, **1980,** 288, 373).
Les composés de l'invention ont été testés sur deux modèles mettant en jeu deux mécanismes différents impliqués dans la dysfonction endothéliale observée en pathologie :
- le premier modèle consiste à induire une inhibition de la relaxation à l'ACh par blocage de l'activité enzymatique (NOS endothéliale) responsable de la production de NO,
- le second modèle consiste à induire un stress oxydant *in vitro* par un système enzymatique générateur d'O₂⁻ (xanthine oxydate -XO et hypoxanthine -Hypo).

### EXEMPLE 41 : Effets protecteurs vasculaires vis-à-vis d'une dysfonction endothéliale induite par un inhibiteur de NOS

L'aorte thoracique de rat Wistar (325-375 g), anesthésié par voie intrapérinétonéale au pentobarbital sodique (30 mg/kg), est prélevée et disséquée en anneaux de 3 mm de longueur. Chaque anneau est suspendu à un capteur de tension isométrique connecté à un système d'acquisition et la tension initiale appliquée est de 2,5 g. La solution physiologique utilisée, thermostatée à 37°C et oxygénée (95 % O₂ + 5 % CO₂) est composée de (en mM) : NaCl 112,0 ; KCl 5,0 ; CaCl₂ 2,5 ; KH₂PO₄ 1,0 ; MgSO₄ 1,2 ; NaHCO₃ 25,0 ; glucose 11,5, Ca-EDTA 0,016.
Après une période de stabilisation de 90 minutes, les préparations sont contractées avec de la phényléphrine (PHE 10⁻⁶M) et relaxées par addition de 10⁻⁵M d'acétylcholine afin de vérifier l'intégrité de la couche endothéliale. Si celle-ci est confirmée, les préparations sont rincées et une concentration de produit à tester est additionnée au milieu suivie de 3.10⁻⁷M de N^{G}-niro-L-arginine (LNA). Les préparations sont à nouveau contractées avec de la phényléphrine et 30 minutes après les relaxations à l'acétylcholine (ACh-10⁻⁸M à 10⁻⁵M) sont évaluées en présence d'indométhacine (10⁻⁵M).
Les valeurs de relaxation sont exprimées en pourcentage par rapport à la contraction maximale à la PHE. Les effets protecteurs des composés vis-à-vis de la dysfonction endothéliale correspondent à la différence entre les pourcentages de relaxation maximale observée en présence ou en absence de produit.
A titre d'exemple, le composé de l'exemple 5 à 10⁻⁷M inhibe de 27 % la dysfonction endothéliale induite par le LNA.

### EXEMPLE 42 : Effets protecteurs vasculaires vis-à-vis d'une dysfonction endothéliale induite par un système générateur de O₂:

Ce protocole, réalisé sur anneaux aortiques de lapins Néo-Zélandais (2,5-3 kg) est comparable au précédent hormis pour les points suivants : la tension initiale appliquée est de 5 g et l'association XO (3 mU/ml) -Hypo (10⁻⁴ M) est utilisée à la place du LNA.
A titre d'exemple, le composé de l'exemple 5 à 10⁻⁷ M inhibe de 17 % la dysfonction endothéliale induite par l'association XO-Hypo.

### EXEMPLE 43 : Implication de la voie du NO dans les effets protecteurs vasculaires détectés : évaluation de la production aortique de GMPc

En diffusant au niveau des cellules musculaires lisses, le NO produit par les cellules endothéliales active la guanylate cyclase soluble ce qui entraîne une augmentation du GMP cyclique responsable de la relaxation.
Ce médiateur a donc été dosé sur les anneaux aortiques de rat afin de démontrer que les effets protecteurs des composés vis-à-vis de la dysfonction endothéliale sont médiés par une augmentation de la disponibilité en NO.
Les anneaux aortiques de rat sont préparés comme précédemment. Les effets d'une incubation de 30 minutes des composés de l'invention à différentes concentrations sont évalués sur la production de GMPc stimulée par l'ACh (10⁻⁵M - 1 minute) en présence de LNA (3x10⁻⁶M). Ces expériences sont réalisées en présence d'isobutyl méthyl xanthine (10⁻⁵M) afin d'éviter la dégradation du GMPc par les phosphodiestérases. Les anneaux sont congelés dans de l'azote liquide et maintenus à -80°C jusqu'au dosage. Le contenu en GMPc est évalué par radio-immunodosage et exprimé par rapport à la quantité de protéines contenues dans le tissu (dosage par la méthode de Bradford).
A titre d'exemple, le composé de l'exemple 5 à 3.10⁻⁷M augmente la production de GMPc stimulée par l'ACh en présence de LNA de 39 %.

### EXEMPLE 44 : Composition pharmaceutique - Comprimé

| Formule de préparation pour 1000 comprimés dosés à 10 mg | |
|---|---|
| Composé de l'exemple 5 | 10 g |
| Hydroxypropylcellulose | 2 g |
| Polyvinylpyrrolidone | 2 g |
| Amidon de blé | 10 g |
| Lactose | 100 g |
| Stéarate de magnésium | 3 g |

## Revendications

1. Composés de formule (I): 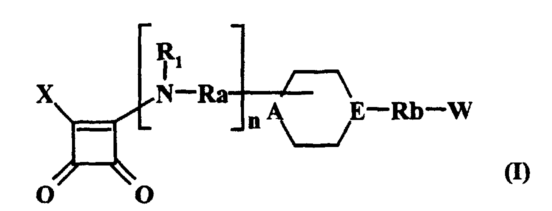 dans laquelle :
**X** représente :
• un hétérocycle, monocyclique ou bicyclique, saturé, partiellement insaturé, ou aromatique, de 5 à 12 chaînons, contenant de 1 à 3 hétéroatomes choisis parmi oxygène, azote, et soufre mais contenant au moins un atome d'azote, ledit hétérocycle étant lié au reste de la molécule par ledit atome d'azote, et ledit hétérocycle étant éventuellement substitué,
• ou un groupement de formule -NR₂R₃ dans laquelle R₂ représente un atome d'hydrogène ou un groupement alkyle (C₁-C₆) linéaire ou ramifié, et R₃ représente un groupement aryle éventuellement substitué, un groupement 1,3-dihydro-2H-benzimidazolyl-2-one ou un groupement alkyle (C₁-C₆) linéaire ou ramifié, subtitué par un hétérocycle, monocyclique ou bicyclique, saturé, partiellement insaturé, ou aromatique, de 5 à 12 chaînons, contenant de 1 à 3 hétéroatomes choisis parmi oxygène, azote, et soufre mais contenant au moins un atome d'azote, ledit hétérocycle étant éventuellement substitué,
**n** représente zéro ou 1,
**R**_{**1**} représente un atome d'hydrogène ou un groupement alkyle (C₁-C₆) linéaire ou ramifié,
**Ra** représente une liaison simple ou une chaîne alkylène (C₁-C₆) linéaire ou ramifiée,
**A** représente un atome d'azote ou un groupement CH, mais A représente uniquement un groupement CH quand Ra représente une liaison simple, et A représente uniquement un atome d'azote quand n représente zéro,
**E** représente un atome d'azote ou un groupement CRe dans lequel Re représente, soit un atome d'hydrogène, soit une liaison avec un atome de carbone de W, étant entendu que l'un au moins des groupements A ou E représente un atome d'azote,
**Rb** représente une liaison simple ou une chaîne alkylène (C₁-C₆) linéaire ou ramifiée, dont un des atomes de carbone est éventuellement remplacé par un atome d'oxygène ou un atome de soufre,
**W** représente un groupement aryle ou hétéroaryle, chacun de ces groupements étant éventuellement substitué,
leurs isomères optiques, leurs hydrates, leurs solvates ainsi que leurs sels d'addition à un acide pharmaceutiquement acceptable,
étant entendu que :
- par groupement aryle, on comprend un groupement choisi parmi phényle, biphényle, bicyclo[42.0]octa-1,3,5-triényle, naphtyle, dihydronaphtyle, tétrahydro-naphtyle, indanyle et indényle,
- par groupement hétéroaryle, on comprend un groupement aryle tel que défini précédemment contenant de 1 à 3 hétéroatomes, identiques ou différents, choisis parmi oxygène, azote et soufre,
- par "éventuellement substitué" affecté aux termes "hétérocycle", "aryle" ou "hétéroaryle", on entend éventuellement substitué par un ou plusieurs groupements, identiques ou différents, choisis parmi halogène, alkyle (C₁-C₆) linéaire ou ramifié, hydroxy, alkoxy (C₁-C₆) linéaire ou ramifié, mercapto, alkylthio (C₁-C₆) linéaire ou ramifié, trihalogénoalkyle (C₁-C₆) linéaire ou ramifié, cyano, nitro, amino, alkylamino (C₁-C₆) linéaire ou ramifié, dialkylamino (C₁-C₆) linéaire ou ramifié et méthylènedioxy,
- par isomères optiques, on comprend les énantiomères et les diastéréoisomères.

2. Composés de formule (I) selon la revendication 1 **caractérisés en ce qu'**ils représentent des composés de formule (IA) : 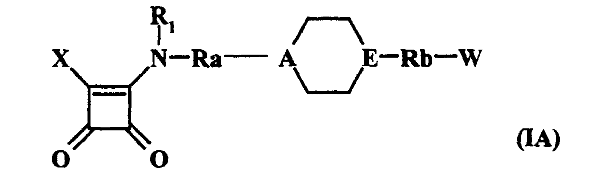 dans laquelle X, R₁, Ra, A, E, Rb et W sont tels que définis dans la formule (I), leurs isomères optiques, leurs hydrates, leurs solvates ainsi que leurs sels d'addition à un acide pharmaceutiquement acceptable.

3. Composés de formule (I) selon la revendication 1 **caractérisés en ce que** X représente un hétérocycle tel que défini dans la formule (I), leurs isomères optiques ainsi que leurs sels d'addition à un acide pharmaceutiquement acceptable.

4. Composés de formule (I) selon la revendication 1 **caractérisés en ce que** X représente un groupement de formule -NR₂R₃ tel que défini dans la formule (I), leurs isomères optiques ainsi que leurs sels d'addition à un acide pharmaceutiquement acceptable.

5. Composés de formule (I) selon l'une quelconque des revendications 1 ou 3 **caractérisés en ce que** X représente un groupement 1-indolinyle éventuellement substitué par un ou plusieurs groupements tels que définis dans la formule (I), leurs isomères optiques ainsi que leurs sels d'addition à un acide pharmaceutiquement acceptable.

6. Composés de formule (I) selon l'une quelconque des revendications 1 ou 3 **caractérisés en ce que** X représente un groupement 2,3-dihydro-1H-pyrrolo [2,3-c]pyridin-1-yle éventuellement substitué par un ou plusieurs groupements tels que définis dans la formule (I), leurs isomères optiques ainsi que leurs sels d'addition à un acide pharmaceutiquement acceptable.

7. Composés de formule (I) selon l'une quelconque des revendications 1 ou 4 **caractérisés en ce que** X représente un groupement de formule -NR₂R₃ dans laquelle R₂ représente un atome d'hydrogène et R₃ représente un groupement phényle éventuellement substitué, leurs isomères optiques ainsi que leurs sels d'addition à un acide pharmaceutiquement acceptable.

8. Composés de formule (I) selon la revendication 1 **caractérisés en ce que** W représente un groupement hétéroaryle, leurs isomères optiques ainsi que leurs sels d'addition à un acide pharmaceutiquement acceptable.

9. Composés de formule (I) selon l'une quelconque des revendications 1 ou 8 **caractérisés en ce que** W représente un groupement benzofuryle ou indolyle, leurs isomères optiques ainsi que leurs sels d'addition à un acide pharmaceutiquement acceptable.

10. Composés de formule (I) selon la revendication 1 **caractérisés en ce que** -Rb-W représente ensemble un groupement aryloxyalkyle (C₁-C₅) linéaire ou ramifié, la partie aryle étant éventuellement substituée, leurs isomères optiques ainsi que leurs sels d'addition à un acide pharmaceutiquement acceptable.

11. Composés de formule (I) selon la revendication 1 qui sont le :
- 3-anilino-4-({2-[4-(phénoxyméthyl)-1-pipéridinyl]éthyl}amino)-3-cyclobutène-1,2-dione et son méthanesulfonate,
- 3-(4-chloroanilino)-4-({2-[4-(phénoxyméthyl)-1-pipéridinyl]éthyl}amino)-3-cyclobutène-1,2-dione et son méthanesulfonate,
- 3-(2,3-dihydro-1*H*-indol-1-yl)-4-({2-[4-(phénoxyméthyl)-1-pipéridinyl]éthyl}amino)-3-cyclobutène-1,2-dione et son méthanesulfonate,
- 3-({1-[2-(1-benzofuran-3-yl)éthyl]-4-pipéridinyl}amino)-4-(4-fluoroanilino)-3-cyclobutène-1,2-dione et son méthanesulfonate,
- 4-{[2-({1-[2-(1-benzofuran-3-yl)éthyl]-4-pipéridinyl}amino)-3,4-dioxo-1-cyclobutèn-1-yl]amino}benzonitrile et son méthanesulfonate,
- 3-[(2-{4-[2-(4-fluorophénoxy)éthyl]-1-pipéridinyl}éthyl)amino]-4-[(2-oxo-2,3-dihydro - 1*H*-benzimidazol-5-yl)amino]-3-cyclobutène-1,2-dione et son méthanesulfonate
- 3-(5-méthoxy-2,3-dihydro-1*H*-pyrrolo[2,3-c]pyridin-1-yl)-4-({2-[4-(phénoxyméthyl)-1-pipéridinyl]éthyl}amino)-3-cyclobutène-1,2-dione et son dichlorhydrate
- 3-[(2-{4-[2-(4-fluorophénoxy)éthyl]-1-pipéridinyl}éthyl)amino]-4-(5-méthoxy-2,3-dihydro-1*H*-pyrrolo[2,3-c]pyridin-1-yl)-3-cyclobutène-1,2-dione et son dichlorhydrate
- 3-[(2-{4-[2-(4-fluorophénoxy)éthyl]-1-pipéridinyl}éthyl)amino]-4-(4,5,6-triméthoxy-2,3-dihydro-1*H*-indol-1-yl)-3-cyclobutène-1,2-dione et son méthanesulfonate
- 3-(6-chloro-2,3-dihydro-1*H*-indol-1-yl)-4-[(2-{4-[2-(4-fluorophénoxy)-éthyl]-1-pipéridinyl}éthyl)amino]-3-cyclobutène-1,2-dione et son méthane sulfonate
- 3-(2,3-dihydro-1*H*-indol-1-yl)-4-[({1-[2-(1*H*-indol-3-yl)éthyl]-3-pipéridinyl}méthyl) amino]-3-cyclobutène-1,2-dione et son chlorhydrate
- 3-(5,6-diméthoxy-2,3-dihydro-1*H*-indol-1-yl)-4-[(2-{4-[2-(4-fluorophénoxy)éthyl]-1-pipéridinyl}éthyl)amino]-3-cyclobutène-1,2-dione et son méthanesulfonate
- 3-[(2-{4-[2-(4-fluorophénoxy)éthyl]-1-pipéridinyl}éthyl)amino]-4-(6-méthoxy-2,3-dihydro-1*H*-indol-1-yl)-3-cyclobutène-1,2-dione et son méthanesulfonate, et le
- 3-(4,5-diméthoxy-2,3-dihydro-1*H*-indol-1-yl)-4-[(2-{4-[2-(4-fluorophénoxy)éthyl]-1-pipéridinyl}éthyl)amino]-3-cyclobutène-1,2-dione et son méthanesulfonate.

12. Procédé de préparation des composés de formule (I) selon la revendication 1, **caractérisé en ce que** soit utilisé comme produit de départ, un composé de formule (II) : 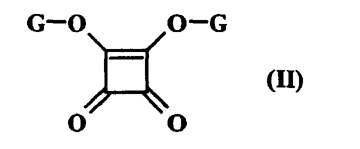 dans laquelle G représente un groupement alkyle (C₁-C₄) linéaire ou ramifié,
composé de formule (II) qui est mis à réagir avec une amine de formule (III) :
**X - H (III)**
dans laquelle X est tel que défini dans la formule (I),
pour conduire au composé de formule (IV) : 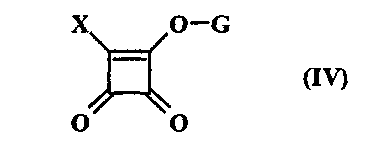 dans laquelle X et G sont tels que définis précédemment,
composé de formule (IV) qui est traité avec un composé de formule (V) : 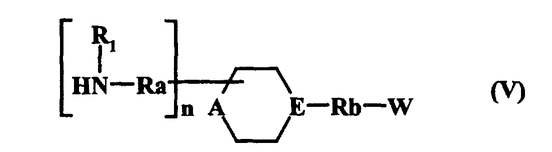 dans laquelle n, R₁, Ra, Rb, A, E et W sont tels que définis dans la formule (I),
pour conduire aux composés de formule (I) telle que définie : 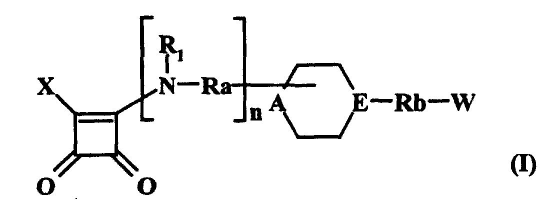 composés de formule (I) que l'on purifie, le cas échéant, selon une technique classique de purification, qui peuvent, si on le désire, être séparés en leurs différents isomères optiques selon une technique classique de séparation et que l'on transforme, si on le souhaite, en leurs sels d'addition à un acide pharmaceutiquement acceptable.

13. Compositions pharmaceutiques contenant comme principe actif au moins un composé de formule (I) selon l'une quelconque des revendications 1 à 11, en combinaison avec un ou plusieurs excipients ou véhicules inertes, non toxiques, pharmaceutiquement acceptables.

14. Compositions pharmaceutiques selon la revendication 13 contenant au moins un principe actif selon l'une quelconque des revendications 1 à 11, utiles pour le traitement des maladies ou des conditions pathologiques dans lesquelles une dysfonction endothéliale est connue.

15. Compositions pharmaceutiques selon la revendication 13 contenant au moins un principe actif selon l'une quelconque des revendications 1 à 11, utiles pour prévenir le développement, l'extension et les complications des lésions athéroscléreuses, pour prévenir les complications vasculaires après pontage vasculaire, dilatation vasculaire, reperméabilisation vasculaire et transplantation cardiaque, ou pour traiter l'ischémie myocardique ou périphérique, l'insuffisance cardiaque et l'hypertension artérielle pulmonaire.

## Patentansprüche

1. Verbindungen der Formel (I): 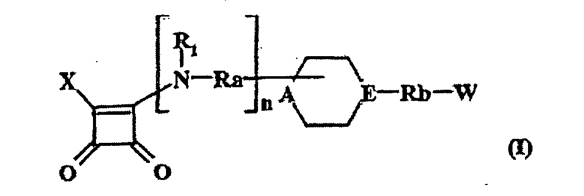 in der:
**X**
• einen monocyclischen oder bicyclischen, gesättigten, teilweise ungesättigten oder aromatischen Heterocyclus mit 5 bis 12 Kettengliedern, der 1 bis 3 Heteroatome ausgewählt aus Sauerstoff, Stickstoff und Schwefel, jedoch mindestens ein Stickstoffatom enthält, welcher Heterocyclus über das Stickstoffatom an den Rest des Moleküls gebunden ist und der Heterocyclus gegebenenfalls substituiert ist,
• oder eine Gruppe der Formel -NR₂R₃, in der R₂ ein Wasserstoffatom oder eine geradkettige oder verzweigte (C₁-C₆)-Alkylgruppe und R₃ eine gegebenenfalls substituierte Arylgruppe, eine 1,3-Dihydro-2H-benzimidazolyl-2-on-gruppe oder eine geradkettige oder verzweigte (C₁-C₆)-Alkylgruppe, die substituiert ist durch einen monocyclischen oder bicyclischen, gesättigten, teilweise ungesättigten oder aromatischen Heterocyclus mit 5 bis 12 Kettengliedern, der 1 bis 3 Heteroatome ausgewählt aus Sauerstoff, Stickstoff und Schwefel enthält, jedoch mindestens ein Stickstoffatom aufweist, wobei der Heterocyclus gegebenenfalls substituiert ist, darstellen, bedeutet,
**n** Null oder 1 bedeutet,
**R**_{**1**} ein Wasserstoffatom oder eine geradkettige oder verzweigte (C₁-C₆)-Alkylgruppe darstellt,
**Ra** eine Einfachbindung oder eine geradkettige oder verzweigte (C₁-C₆)-Alkylenkette bedeutet,
**A** ein Stickstoffatom oder eine Gruppe CH bedeutet, wobei jedoch A nur dann eine CH-Gruppe darstellt, wenn Ra eine Einfachbindung bedeutet, und A nur dann ein Stickstoffatom darstellt, wenn n den Wert Null besitzt,
**E** ein Stickstoffatom oder eine Gruppe CRe bedeutet, worin Re entweder ein Wasserstoffatom oder eine Bindung mit einem Kohlenstoffatom von W darstellt,
mit der Maßgabe, daß mindestens eine der Gruppen A oder E ein Stickstoffatom bedeutet,
**Rb** eine Einfachbindung oder eine geradkettige oder verzweigte (C₁-C₆)-Alkylenkette bedeutet, wobei eines der Kohlenstoffatome gegebenenfalls durch ein Sauerstoffatom oder ein Schwefelatom ersetzt ist,
**W** eine Aryl- oder Heteroarylgruppe beduetet, wobei jede dieser Gruppen gegebenenfalls substituiert ist,
deren optische Isomere, deren Hydrate, deren Solvate sowie deren Additionssalze mit einer pharmazeutisch annehmbaren Säure,
wobei es sich versteht, daß man:
- unter einer Arylgruppe eine Gruppe versteht, die ausgewählt ist aus Phenyl, Biphenyl, Bicyclo[4.2.0]octa-1,3,5-trienyl, Naphthyl, Dihydronaphthyl, Tetrahydronaphthyl, Indanyl und Indenyl,
- unter einer Heteroarylgruppe eine Arylgruppe versteht, wie sie oben definiert ist, die 1 bis 3 gleichartige oder verschiedenartige Heteroatome ausgewählt aus Sauerstoff, Stickstoff und Schwefel enthält,
- unter "gegebenenfalls substituiert" in bezug auf die Begriffe "Heterocyclus", "Aryl" oder "Heteroaryl" eine eventuelle Substitution durch eine oder mehrere gleichartige oder verschiedenartige Gruppen versteht, ausgewählt aus Halogen, geradkettigem oder verzweigtem (C₁-C₆)-Alkyl, Hydroxy, geradkettigem oder verzweigtem (C₁-C₆)-Alkoxy, Mercapto, geradkettigem oder verzweigtem (C₁-C₆)-Alkylthio, geradkettigem oder verzweigtem (C₁-C₆)-Trihalogenalkyl, Cyano, Nitro, Amino, geradkettigem oder verzweigtem (C₁-C₆)-Alkylamino, geradkettigem oder verzweigtem Di-(C₁-C₆)-alkylamino und Methylendioxy,
- und unter optischen Isomeren die Enantiomeren und die Diastereoisomeren versteht.

2. Verbindungen der Formel (I) nach Anspruch 1, **dadurch gekennzeichnet, daß** sie der Formel (IA) entsprechen: 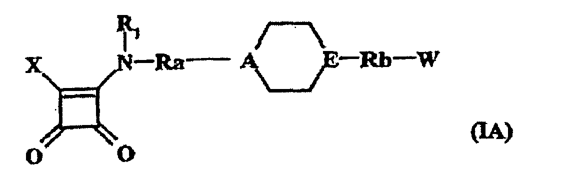 in der X, R₁, Ra, A, E, Rb und W die bezüglich der Formel (I) angegebenen Bedeutungen besitzen, deren optische Isomere, deren Hydrate, deren Solvate sowie deren Additionssalze mit einer pharmazeutisch annehmbaren Säure.

3. Verbindungen der Formel (I) nach Anspruch 1, **dadurch gekennzeichnet, daß** X einen Heterocyclus bedeutet, wie er bezüglich der Formel (I) definiert worden ist, deren optische Isomere sowie deren Additionssalze mit einer pharmazeutisch annehmbaren Säure.

4. Verbindungen der Formel (I) nach Anspruch 1, **dadurch gekennzeichnet, daß** X eine Gruppe der Formel -NR₂R₃ darstellt, wie sie bezüglich der Formel (I) definiert worden ist, deren optische Isomere sowie deren Additionssalze mit einer pharmazeutisch annehmbaren Säure.

5. Verbindungen der Formel (I) nach einem der Ansprüche 1 oder 3, **dadurch gekennzeichnet, daß** X eine 1-Indolinylgruppe bedeutet, die gegebenenfalls durch eine oder mehrere Gruppen substituiert ist, wie sie bezüglich der Formel (I) definiert worden sind, deren optische Isomere sowie deren Additionssalze mit einer pharmazeutisch annehmbaren Säure.

6. Verbindungen der Formel (I) nach einem der Ansprüche 1 oder 3, **dadurch gekennzeichnet, daß** X eine 2,3-Dihydro-1H-pyrrolo[2,3-c]pyridin-1-yl-gruppe bedeutet, die gegebenenfalls durch eine oder mehrere Gruppen substituiert ist, wie sie bezüglich der Formel (I) definiert worden ist, deren optische Isomere sowie deren Additionssalze mit einer pharmazeutisch annehmbaren Säure.

7. Verbindungen der Formel (I) nach einem der Ansprüche 1 oder 4, **dadurch gekennzeichnet, daß** X eine Gruppe der Formel -NR₂R₃ darstellt, in der R₂ ein Wasserstoffatom und R₃ eine gegebenenfalls substituierte Phenylgruppe bedeutet, deren optische Isomere sowie deren Additionssalze mit einer pharmazeutisch annehmbaren Säure.

8. Verbindungen der Formel (I) nach Anspruch 1, **dadurch gekennzeichnet, daß** W eine Heteroarylgruppe bedeutet, deren optische Isomere sowie deren Additionssalze mit einer pharmazeutisch annehmbaren Säure.

9. Verbindungen der Formel (I) nach einem der Ansprüche 1 oder 8, **dadurch gekennzeichnet, daß** W eine Benzofuryl- oder Indolylgruppe bedeutet, deren optische Isomere sowie deren Additionssalze mit einer pharmazeutisch annehmbaren Säure.

10. Verbindungen der Formel (I) nach Anspruch 1, **dadurch gekennzeichnet, daß** -Rb-W gemeinsam eine geradkettige oder verzweigte Aryloxy-(C₁-C₅)-alkylgruppe bedeuten, wobei der Arylrest gegebenenfalls substituiert ist, deren optische Isomere sowie deren Additionssalze mit einer pharmazeutisch annehmbaren Säure.

11. Verbindungen der Formel (I) nach Anspruch 1, nämlich:
- 3-Anilino-4-({2-[4-(phenoxymethyl)-1-piperidinyl]-ethyl}-amino)-3-cyclobuten-1,2-dion und dessen Methansulfonat,
- 3-(4-Chloranilino)-4-({2-[4-(phenoxymethyl)-1-piperidinyl]-ethyl}-amino)-3-cyclobuten-1,2-dion und dessen Methansulfonat,
- 3-(2,3-Dihydro-1*H*-indol-1-yl)-4-({2-[4-(phenoxymethyl)-1-piperidinyl]-ethyl)-amino)-3-cyclobuten-1,2-dion und dessen Methansulfonat,
- 3-({1-[2-(1-Benzofuran-3-yl)-ethyl]-4-piperidinyl}-amino)-4-(4-fluoranilino)-3-cyclobuten-1,2-dion und dessen Methansulfonat,
- 4-{[2-({1-[2-(1-Benzofuran-3-yl)-ethyl]-4-piperidinyl}-amino)-3,4-dioxo-1-cyclobuten-1-yl]-amino}-benzonitril und dessen Methansulfonat,
- 3-[(2-{4-[2-(4-Fluorphenoxy)-ethyl)-1-piperidinyl}-ethyl)-amino]-4-[(2-oxo-2,3-dihydro-1*H*-benzimidazol-5-yl)-amino]-3-cyclobuten-1,2-dion und dessen Methansulfonat,
- 3-(5-Methoxy-2,3-dihydro-1*H*-pyrrolo[2,3-c]pyridin-1-yl)-4-({2-[4-(phenoxymethyl)-1-piperidinyl]-ethyl}-amino)-3-cyclobuten-1,2-dion und dessen Dihydrochlorid,
- 3-[(2-{4-[2-(4-Fluorphenoxy)-ethyl]-1-piperidinyl}-ethyl)-amino]-4-(5-methoxy-2,3-dihydro-1*H*-pyrrolo[2,3-c]pyridin-1-yl-3-cyclobuten-1,2-dion und dessen Dihydrochlorid,
- 3-[(2-{4-[2-(4-Fluorphenoxy)-ethyl]-1-piperidinyl}-ethyl)-amino]-4-(4,5,6-trimethoxy-2,3-dihydro-1*H*-indol-1-yl)-3-cyclobuten-1,2-dion und dessen Methansulfonat,
- 3-(6-Chlor-2,3-dihydro-1*H*-indol-1-yl)-4-[(2-{4-[2-(4-fluorphenoxy)-ethyl]-1-piperidinyl}-ethyl)-amino]-3-cyclobuten-1,2-dion und dessen Methansulfonat,
- 3-(2,3-Dihydro-1*H*-indol-1-yl)-4-[({1-[2-(1*H*-indol-3-yl)-ethyl]-3-piperidinyl}-methyl)-amino]-3-cyclobuten-1,2-dion und dessen Hydrochlorid,
- 3-(5,6-Dimethoxy-2,3-dihydro-1*H*-indol-1-yl)-4-[(2-{4-[2-(4-fluorphenoxy)-ethyl]-1-piperidinyl}-ethyl)-amino]-3-cyclobuten-1,2-dion und dessen Methansulfonat.
- 3-[(2-{4-[2-(4-Fluorphenoxy)-ethyl]-1-piperidinyl}-ethyl)-amino]-4-(6-methoxy-2,3-dihydro-1*H*-indol-1-yl)-3-cyclobuten-1,2-dion und dessen Methansulfonat sowie
- 3-(4,5-Dimethoxy-2,3-dihydro-1*H*-indol-1-yl)-4-[(2-{4-[2-(4-fluorphenoxy)-ethyl]-1-piperidinyl}-ethyl)-amino]-3-cyclobuten-1,2-dion und dessen Methansulfonat.

12. Verfahren zur Herstellung der Verbindungen der Formel (I) nach Anspruch 1, **dadurch gekennzeichnet, daß** man als Ausgangsprodukt eine Verbindung der Formel (II) verwendet: 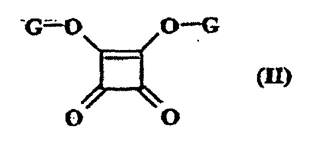 in der G eine geradkettige oder verzweigte (C₁-C₄)-Alkylgruppe bedeutet, welche Verbindung der Formel (II) mit einem Amin der Formel (III) umgesetzt wird:
**X - H (III)**
in der X die bezüglich der Formel (I) angegebenen Bedeutungen besitzt,
zur Bildung der Verbindung der Formel (IV): 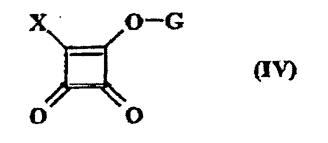 in der X und G die oben angegebenen Bedeutungen besitzen,
welche Verbindung der Formel (IV) mit einer Verbindung der Formel (V) behandelt wird: 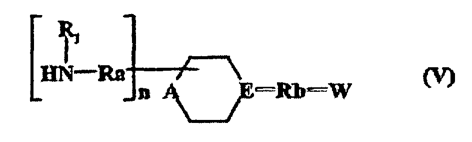 in der n, R₁, Ra, Rb, A, E und W die bezüglich der Formel (I) angegebenen Bedeutungen besitzen,
zur Bildung der Verbindungen der Formel (I): 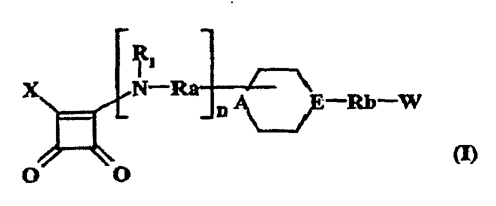 welche Verbindungen der Formel (I) man gegebenenfalls mit Hilfe einer klassischen Reinigungsmethode reinigt, gewünschtenfalls mit Hilfe einer klassischen Trennmethode in ihre verschiedenen optischen Isomeren auftrennt und gewünschtenfalls in ihre Additionssalze mit einer pharmazeutisch annehmbaren Säure umwandelt.

13. Pharmazeutische Zubereitungen, enthaltend als Wirkstoff mindestens eine Verbindung der Formel (I) nach einem der Ansprüche 1 bis 11 in Kombination mit einem oder mehreren inerten, nichttoxischen, pharmazeutisch annehmbaren Trägermaterialien oder Bindemitteln.

14. Pharmazeutische Zubereitungen nach Anspruch 13, enthaltend mindestens einen Wirkstoff nach einem der Ansprüche 1 bis 11, für die Behandlung von Erkrankungen oder pathologischen Zuständen, bei denen eine endotheliale Dysfunktion bekannt ist.

15. Pharmazeutische Zubereitungen nach Anspruch 13, enthaltend mindestens einen Wirkstoff nach einem der Ansprüche 1 bis 11, für die Vorbeugung der Entwicklung. Ausbreitung und von Komplikationen von arteriosklerösen Verletzungen, zur Vorbeugung von Gefäßkomplikationen nach Gefäßüberbrückung, der Gefäβerweiterung, der Gefäβ-Repermeabilisierung und der Herztransplantation oder zur Behandlung der myokardischen oder peripheren Ischämie, der Herzinsuffizienz und der Lungenarterienhypertension.

## Claims

1. Compounds of formula (I) : 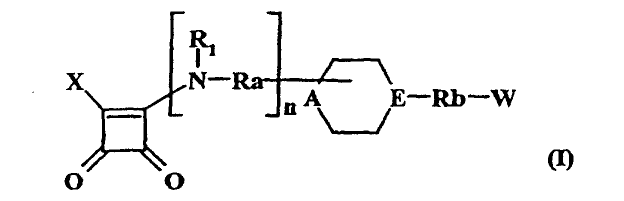 wherein :
**X** represents:
• a monocyclic or bicyclic, saturated, partially unsaturated or aromatic heterocycle, having from 5 to 12 ring members, containing from 1 to 3 hetero atoms selected from oxygen, nitrogen and sulphur but containing at least one nitrogen atom, said heterocycle being bonded to the remainder of the molecule by said nitrogen atom, and said heterocycle being optionally substituted,
• or a group of formula -NR₂R₃ wherein R₂ represents a hydrogen atom or a linear or branched (C₁-C₆)alkyl group, and R₃ represents an optionally substituted aryl group, a 1,3-dihydro-2H-benzimidazolyl-2-one group or a linear or branched (C₁-C₆)alkyl group, substituted by a monocyclic or bicyclic, saturated, partially unsaturated or aromatic heterocycle, having from 5 to 12 ring members, containing from 1 to 3 hetero atoms selected from oxygen, nitrogen and sulphur but containing at least one nitrogen atom, said heterocycle being optionally substituted,
**n** is zero or 1,
**R**_{**1**} represents a hydrogen atom or a linear or branched (C₁-C₆)alkyl group,
**Ra** represents a single bond or a linear or branched (C₁-C₆)alkylene chain,
**A** represents a nitrogen atom or a CH group, but A represents only a CH group when Ra represents a single bond, and A represents only a nitrogen atom when n is zero,
**E** represents a nitrogen atom or a CRe group wherein Re represents a hydrogen atom or a bond with a carbon atom of W,
with the proviso that at least one of the groups A and E represents a nitrogen atom,
**Rb** represents a single bond or a linear or branched (C₁-C₆)alkylene chain, one of the carbon atoms of which is optionally replaced by an oxygen atom or a sulphur atom,
**W** represents an aryl or heteroaryl group, each of those groups being optionally substituted,
their optical isomers, their hydrates, their solvates and addition salts thereof with a pharmaceutically acceptable acid,
it being understood that:
- "aryl group" is understood to mean a group selected from phenyl, biphenyl, bicyclo[4.2.0]octa-1,3,5-trienyl, naphthyl, dihydronaphthyl, tetrahydronaphthyl, indanyl and indenyl,
- "heteroaryl group" is understood to mean an aryl group as defined above containing from 1 to 3 identical or different hetero atoms selected from oxygen, nitrogen and sulphur,
- "optionally substituted" applied to the terms "heterocycle", "aryl" or "heteroaryl" is understood to mean optionally substituted by one or more identical or different groups selected from halogen, linear or branched (C₁-C₆)alkyl, hydroxy, linear or branched (C₁-C₆)alkoxy, mercapto, linear or branched (C₁-C₆)alkylthio, linear or branched (C₁-C₆)trihaloalkyl, cyano, nitro, amino, linear or branched (C₁-C₆)alkylamino, dialkylamino in which each alkyl moiety has from 1 to 6 carbon atoms and is linear or branched, and methylenedioxy,
- "optical isomers" are understood to mean the enantiomers and diastereoisomers.

2. Compounds of formula (I) according to claim 1, **characterised in that** they represent compounds of formula (IA) : 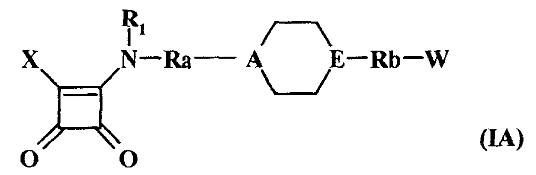 wherein X, R₁, Ra, A, E, Rb and W are as defined for formula (I), their optical isomers, their hydrates, their solvates and addition salts thereof with a pharmaceutically acceptable acid.

3. Compounds of formula (I) according to claim 1, **characterised in that** X represents a heterocycle as defined for formula (I), their optical isomers and addition salts thereof with a pharmaceutically acceptable acid.

4. Compounds of formula (I) according to claim 1, **characterised in that** X represents a group of formula -NR₂R₃ as defined for formula (I), their optical isomers and addition salts thereof with a pharmaceutically acceptable acid.

5. Compounds of formula (I) according to either claim 1 or claim 3, **characterised in that** X represents a 1-indolinyl group optionally substituted by one or more groups as defined for formula (I), their optical isomers and addition salts thereof with a pharmaceutically acceptable acid.

6. Compounds of formula (I) according to either claim 1 or claim 3, **characterised in that** X represents a 2,3-dihydro-1H-pyrrolo[2,3-c]pyrid-1-yl group optionally substituted by one or more groups as defined for formula (I), their optical isomers and addition salts thereof with a pharmaceutically acceptable acid.

7. Compounds of formula (I) according to either claim 1 or claim 4, **characterised in that** X represents a group of formula -NR₂R₃ wherein R₂ represents a hydrogen atom and R₃ represents an optionally substituted phenyl group, their optical isomers and addition salts thereof with a pharmaceutically acceptable acid.

8. Compounds of formula (I) according to claim 1, **characterised in that** W represents a heteroaryl group, their optical isomers and addition salts thereof with a pharmaceutically acceptable acid.

9. Compounds of formula (I) according to either claim 1 or claim 8, **characterised in that** W represents a benzofuryl or indolyl group, their optical isomers and addition salts thereof with a pharmaceutically acceptable acid.

10. Compounds of formula (I) according to claim 1, **characterised in that** -Rb-W together represent an aryloxyalkyl group in which the alkyl moiety has from 1 to 5 carbon atoms and is linear or branched and the aryl moiety is optionally substituted, their optical isomers and addition salts thereof with a pharmaceutically acceptable acid.

11. Compounds of formula (I) according to claim 1 which are :
- 3-anilino-4-({2-[4-(phenoxymethyl)-1-piperidyl]ethyl}amino)-3-cyclobutene-1,2-dione and its methanesulphonate,
- 3-(4-chloroanilino)-4-({2-[4-(phenoxymethyl)-1-piperidyl]ethyl}amino)-3-cyclobutene-1,2-dione and its methanesulphonate,
- 3-(2,3-dihydro-1*H*-indol-1-yl)-4-({2-[4-(phenoxymethyl)-1-piperidyl]ethyl}amino)-3-cyclobutene-1,2-dione and its methanesulphonate,
- 3-({1-[2-(1-benzofuran-3-yl)ethyl]-4-piperidyl}amino)-4-(4-fluoroanilino)-3-cyclobutene-1,2-dione and its methanesulphonate,
- 4-{[2-({1-[2-(1-benzofuran-3-yl)ethyl]-4-piperidyl}amino)-3,4-dioxo-1-cyclobuten-1-yl]amino}benzonitrile and its methanesulphonate,
- 3-[(2-{4-[2-(4-fluorophenoxy)ethyl]-1-piperidyl}ethyl)amino]-4-[(2-oxo-2,3-dihydro-1*H*-benzimidazol-5-yl)amino]-3-cyclobutene-1,2-dione and its methanesulphonate,
- 3-(5-methoxy-2,3-dihydro-1*H*-pyrrolo[2,3-c]pyrid-1-yl)-4-({2-[4-(phenoxymethyl)-1-piperidyl]ethyl}amino)-3-cyclobutene-1,2-dione and its dihydrochloride,
- 3-[(2-{4-[2-(4-fluorophenoxy)ethyl]-1-piperidyl}ethyl)amino]-4-(5-methoxy-2,3-dihydro-1*H*-pyrrolo[2,3-c]pyrid-1-yl)-3-cyclobutene-1,2-dione and its dihydrochloride,
- 3-[(2-{4-[2-(4-fluorophenoxy)ethyl]-1-piperidyl}ethyl)amino]-4-(4,5,6-trimethoxy-2,3-dihydro-1*H*-indol-1-yl)-3-cyclobutene-1,2,dione and its methanesulphonate,
- 3-(6-chloro-2,3-dihydro-1*H*-indol-1-yl)-4-[(2-{4-[2-(4-fluorophenoxy)-ethyl]-1-piperidyl}ethyl)amino]-3-cyclobutene-1,2-dione and its methanesulphonate,
- 3-(2,3-dihydro-1*H*-indol-1-yl)-4-[({1-[2-(1*H*-indol-3-yl)ethyl]-3-piperidyl}methyl)-amino]-3-cyclobutene-1,2-dione and its hydrochloride,
- 3-(5,6-dimethoxy-2,3-dihydro-1*H*-indol-1-yl)-4-[(2-{4-[2-(4-fluorophenoxy)ethyl]-1-piperidyl}ethyl)amino]-3-cyclobutene-1,2-dione and its methanesulphonate,
- 3-[(2-{4-[2-(4-fluorophenoxy)ethyl]-1-piperidyl}ethyl)amino]-4-(6-methoxy-2,3-dihydro-1*H*-indol-1-yl)-3-cyclobutene-1,2-dione and its methanesulphonate, and
- 3-(4,5-dimethoxy-2,3-dihydro-1*H*-indol-1-yl)-4-[(2-{4-[2-(4-fluorophenoxy)ethyl]-1-piperidyl}ethyl)amino]-3-cyclobutene-1,2-dione and its methanesulphonate.

12. Process for the preparation of compounds of formula (I) according to claim 1, **characterised in that** there is used as starting material a compound of formula (II) : 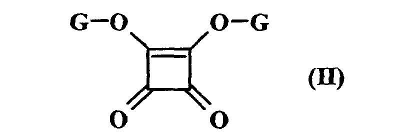 wherein G represents a linear or branched (C₁-C₄)alkyl group,
which compound of formula (II) is reacted with an amine of formula (III):
**X** - **H (III)**
wherein X is as defined for formula (I),
to yield a compound of formula (IV) : 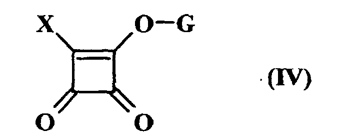 wherein X and G are as defined hereinbefore,
which compound of formula (IV) is treated with a compound of formula (V) : 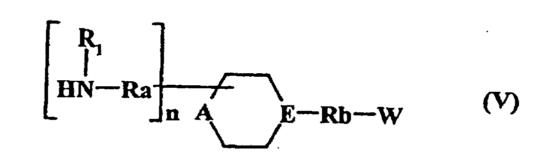 wherein n, R₁, Ra, Rb, A, E and W are as defined for formula (I),
to yield compounds of formula (I) as defined : 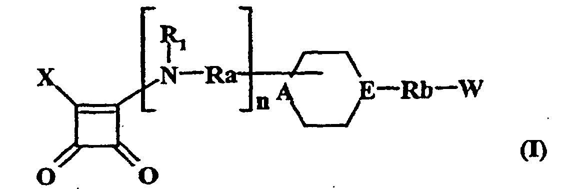 which compounds of formula (I) are purified, if necessary, according to a conventional purification technique, may, if desired, be separated into their different optical isomers according to a conventional separation technique, and are converted, if desired, into addition salts thereof with a pharmaceutically acceptable acid.

13. Pharmaceutical compositions comprising as active ingredient at least one compound of formula (I) according to any one of claims 1 to 11, in combination with one or more inert, non-toxic, pharmaceutically acceptable excipients or carriers.

14. Pharmaceutical compositions according to claim 13 comprising at least one active ingredient according to any one of claims 1 to 11 for use in the treatment of diseases or pathological conditions in which endothelial dysfunction is known.

15. Pharmaceutical compositions according to claim 13 comprising at least one active ingredient according to any one of claims 1 to 11 for use in preventing the development, extension and complications of atherosclerotic lesions, in preventing vascular complications after vascular bypass, vascular dilatation, vascular repermeabilisation and heart transplantation, or in the treatment of myocardial or peripheral ischaemia, cardiac insufficiency and pulmonary arterial hypertension.
